# EUROPÄISCHE PATENTSCHRIFT

(11) **EP 3 459 874 B1**
(45) Veröffentlichungstag und Bekanntmachung des Hinweises auf die Patenterteilung: **28.06.2023**
(21) Anmeldenummer: 17192606.6
(22) Anmeldetag: 22.09.2017
(51) Int. Cl.: C09D 7/40, D06N 3/06, D06N 3/08, D06N 3/18, D06N 5/00, B32B 3/26, B32B 3/28, B32B 5/08, B32B 27/30, B32B 27/32, C12M 1/107, B32B 27/12, B32B 27/20, E04H 7/06, E04D 5/10, C09D 5/00, D06N 3/00, E04H 7/02

(54) **FLÄCHIGES GEBILDE FÜR EINEN BIOGASSPEICHER**
FLAT ARTICLE FOR BIOGAS STORAGE
STRUCTURE PLANE POUR UN ACCUMULATEUR DE BIOGAZ

(43) Veröffentlichungstag der Anmeldung: 27.03.2019
(73) Patentinhaber: Sattler PRO-TEX GmbH, 8077 Gössendorf (AT)
(72) Erfinder: Hauser, Julia, 8042 Graz (AT); Pichler, Michael, 8046 Graz (AT); Gradnig, Günther, 9400 St. Johann (AT)
(74) Vertreter: Wirnsberger & Lerchbaum Patentanwälte OG

(56) Entgegenhaltungen:
- EP-A2- 2 746 385
- DE-A1- 3 102 169
- DE-A1- 3 501 474
- DE-U- 7 830 648
- US-A1- 2004 180 593
- US-A1- 2005 106 967

## Beschreibung

Die Erfindung betrifft ein flächiges Gebilde, insbesondere für eine Dachkonstruktion eines Biogasspeichers, umfassend ein Gewebe, welches auf zumindest einer Seite zumindest bereichsweise von einer Kunststoffschicht umgeben ist, wobei auf der Kunststoffschicht eine Lackschicht aufgebracht ist. Dokument US 2005/106967 A1 zeigt ein flächiges Gebilde, umfassend ein Gewebe, welches auf einer Seite von einer Kunststoffschicht umgeben ist, wobei auf der Kunststoffschicht eine Lackschicht aufgebracht ist, wobei die Lackschicht neben einem Lackharz beigemengte Partikel enthält, wobei eine durchschnittliche Größe der Partikel in der Lackschicht mehr als 5 µm beträgt und die Lackschicht mehr als 5 Gew.-% Partikel enthält.

Des Weiteren betrifft die Erfindung eine Verwendung eines flächigen Gebildes.

Aus dem Stand der Technik sind Gasspeicher bekannt, die insbesondere für die Speicherung von Gasen Einsatz finden, die durch Fermentation aus Material biogenen Ursprungs gewonnen werden. Bei den derart verwerteten (Abfall-)Produkten kann es sich beispielsweise um Ausscheidungen von Nutztieren handeln. In jüngerer Zeit werden auch aus Lebensmittelabfällen im Sinne einer nachhaltigen Verwertung Gase wie Methangas gewonnen, die anschließend für eine thermische Nutzung zur Verfügung stehen. Zur Zwischenspeicherung werden die Gase häufig in Gasspeichern vorrätig gehalten.

Gasspeicher zur Speicherung von Biogasen sind oftmals als sogenannte Membrangasspeicher ausgebildet. Entsprechende Gasspeicher weisen ein inneres Volumen auf, das zumindest bereichsweise von einer Gasspeicherfolie bzw. -membran umgeben ist. Eine solche Membran kann aus verschiedenen Materialien gebildet sein, beispielsweise Kunststoffen wie Polyvinylchlorid (PVC) oder auch einem beschichteten Gewebe. Ein entsprechender Gasspeicher kann beispielsweise so aufgebaut sein, dass dieser in Draufsicht eine kreisförmige Behälterwand aufweist, auf welcher eine Membran durch Klemmringe angebracht ist, wodurch ein Innenraum gebildet wird. In diesem Innenraum wird Biogas gebildet oder in diesen eingespeist, wobei sich die hierfür gasdicht ausgebildete Membran ausdehnen kann. Möglich sind dabei auch sogenannte Doppelmembranen, wobei die Innenmembran in der beschriebenen Weise gasdicht und ausdehnbar ist, sodass eine variable Reaktion in Bezug auf gebildetes oder eingespeistes Gas möglich ist, während die Außenmembran weitgehend konstant bleibt und vornehmlich dazu dient, die Innenmembran gegen Witterung und andere Einflüsse zu schützen.

Biogasspeicher müssen nicht unbedingt mit einer runden Behälterwand ausgebildet sein, sondern können grundsätzlich auch mit einer relativ schlanken, bodennahen Grundkonstruktion auskommen, auf welcher eine Membran befestigt ist. In einem solchen Fall kann ein Biogasspeicher insbesondere kugelförmig ausgebildet sein.

Biogasspeicher können je nach Ausbildung und gewünschtem Volumen durchaus beträchtliche Durchmesser von 10 m bis 40 m aufweisen. Die entsprechenden Biogasspeicher sind daher bei der erforderlichen Aufstellung im freien Gelände üblicherweise über große Distanzen gut sichtbar. Das äußere Erscheinungsbild wird dabei auch von der Membran bzw. einem flächigen Gebilde für die Dachkonstruktion oder gegebenenfalls auch für den gesamten Biogasspeicher signifikant mitbestimmt. Die Farbe der Membran bzw. Folie spielt dabei selbstredend eine große Rolle. Hierbei kommen beispielsweise grüne oder in Grüntönen gefärbte Membranen bzw. Folien zum Einsatz, wodurch grundsätzlich eine optisch zurückhaltende Eingliederung eines Biogasspeichers in ein natürliches Landschaftsbild möglich ist. Ästhetisch besonders anmutig und daher aus solchen Gesichtspunkten bevorzugt sind jedoch Biogasspeicher, die mit einer hellen bzw. weißen Außenhaut ausgebildet sind, da dies in keiner Weise auf die Verarbeitung von biogenen Abfällen und daraus gebildete Gase schließen lässt, welche aufgrund eines Geruchs negative Assoziationen auslösen können.

Membranen für Biogasspeicher können beispielsweise auf Basis eines Gewebes gebildet sein, das beidseitig von einem Kunststoff wie PVC umgeben ist. Auf dem Kunststoff ist in der Regel ein Lack abgeschieden, sodass sich ein mehrschichtiger Aufbau ergibt. Derartige Membranen bzw. flächige Gebilde werden auch zur Herstellung von Biogasspeichern eingesetzt, bei welchen zumindest Teile der Außenhülle aus der Membran gebildet werden und wobei die Membran vorzugsweise in weißer Farbe oder zumindest mit einem hellen Farbton wie grau oder grün vorliegt, sodass die angesprochene anmutige Erscheinung gegeben ist.

Bei der Verwendung von hellen Membranen, insbesondere mit weißer Farbe oder ins Weiße gehendem Farbton hat sich gezeigt, dass im Laufe der Zeit eine Verfärbung der Membran auftritt, die ins Bräunliche geht und daher zu einem besonders nachteiligen Erscheinungsbild führt.

Hier setzt die Erfindung an. Aufgabe der Erfindung ist es, eine Membran bzw. ein flächiges Gebilde der eingangs genannten Art anzugeben, bei welchem der vorstehende Nachteil vermieden oder zumindest verringert ist.

In einem weiteren Aspekt betrifft die Erfindung die Verwendung eines flächigen Gebildes.

Die Aufgabe wird durch ein flächiges Gebild nach Anspruch 1 gelöst.

Ein erfindungsgemäßes flächiges Gebilde bzw. eine solche Membran bringt bei einem Einsatz als Außenhülle eines Biogasspeichers oder zumindest Teil derselben den Vorteil, dass sich die Außenhülle im Laufe der Zeit nicht oder nur unmerklich verfärbt. Umfangreiche Versuche haben gezeigt, dass sich aufgrund der Fermentation biogener Abfälle im Bereich des Gewebes dunkle Abscheidungen bilden können. Die Zusammensetzung dieser Abscheidungen ist nicht bekannt, es könnte sich jedoch um höhermolekulare organische Verbindungen handeln, die sich durch Reaktion und/oder Kondensation am Gewebe bilden. Ist nun eine außenseitige, also eine an der dem Innenvolumen abgewandten Seite der Außenhülle, Lackschicht mit Partikeln ausgebildet, die beispielsweise einem herkömmlich eingesetzten Klarlack beigemengt werden, kommt es gemäß Untersuchungen durchaus auch zur Bildung dieser Abscheidungen im Bereich des Gewebes, allerdings werden diese durch die Lackschicht bzw. die darin enthaltenen Partikel abgedeckt. Das flächige Gebilde bzw. die Membran kann aber nach wie vor weiß (also mit einem Klarlack mit den Partikeln und einem weißen Gewebe) ausgebildet sein.

Die Partikel sind zumindest überwiegend als Plättchen ausgebildet. Metallpigmente stellen Partikel dar, die im Rahmen der Erfindung eingesetzt werden können.

Wiewohl nicht zwingend, kann das Gewebe beidseitig von einer Kunststoffschicht umgeben sein. Dabei ist vorzugsweise vorgesehen, um eine Herstellung möglichst einfach zu halten, dass das Gewebe zu beiden Seiten von demselben Material umgeben ist. Als Material kommen beliebige Kunststoffe zum Einsatz, insbesondere Polypropylen (PP), Niederdruckpolyethylen (LDPE), PVC oder Varianten davon wie PP/PVC. Eine Dicke der einzelnen Kunststoffschichten kann dabei im Bereich von 100 µm bis 700 µm, bevorzugt 200 µm bis 500 µm, liegen.

In diesem Zusammenhang kann insbesondere auch vorgesehen sein, dass auf jeder Kunststoffschicht eine Lackschicht aufgebracht ist. Für die Lackschichten kann für eine einfache Herstellung dasselbe Material für die einzelnen Lackschichten verwendet werden. Bevorzugt ist es jedoch, dass für eine innenseitige Lackschicht eine partikelfreie Lackschicht vorgesehen ist, da die innenseitige Lackschicht bezüglich der gewünschten Wirkungen nicht oder zumindest weniger relevant ist und dann kostengünstig bereitgestellt werden kann. Hierfür eignet sich z. B. ein transparenter Lack oder ein Klarlack, wie dieser bisher außenseitig eingesetzt wurde.

Die Lackschicht enthält mehr als 5 Gewichtsprozent (Gew.-%) Partikel, vorzugsweise mehr als 10 Gew.-% Partikel, insbesondere 12 Gew.-% bis 20 Gew.-% Partikel. Je mehr Partikel in der Lackschicht anteilsmäßig vorhanden sind, umso besser werden sich im Laufe der Zeit am oder im Bereich des Gewebes oder gegebenenfalls auch in den das Gewebe umgebenden Kunststoffschichten bildende Ablagerungen durch die nach außen hin sichtbare Lackschicht verdeckt. Damit bleibt das Erscheinungsbild der Membran bzw. des flächigen Gebildes im Einsatz auch dann erhalten, wenn sich die an sich unerwünschten Ablagerungen bzw. Abscheidungen bilden. Damit der Lack selbst aber gut verarbeitbar bleibt und auch dauerhaft am darunterliegenden Kunststoff halten kann, ist es zweckmäßig, wenn ein Anteil der Partikel auf maximal 20 Gew.-% begrenzt ist.

Über eine durchschnittliche Partikelgröße der Partikel in der Lackschicht können die Eigenschaften des flächigen Gebildes bzw. einer Membran weiter optimiert werden. Die in der Lackschicht vorhandenen Partikel weisen eine durchschnittliche Partikelgröße von mehr als 5 µm, vorzugsweise mehr als 10 µm, insbesondere 12 µm bis 25 µm, auf. Für die Bestimmung der durchschnittlichen Partikelgröße werden die maximalen Durchmesser der Partikel gemittelt. Mit einer größeren durchschnittlichen Partikelgröße der Partikel in der Lackschicht können darunterliegende Abscheidungen besser abgedeckt werden. Ist eine durchschnittliche Partikelgröße allerdings zur groß, besteht die Gefahr, dass sich innerhalb der relativ dünnen Lackschicht, die üblicherweise eine Dicke von 0,001 mm bis 0,005 mm aufweist, Inhomogenitäten ausbilden, was für das Erscheinungsbild einer Membran bzw. allgemein des flächigen Gebildes nachteilig wäre.

Die Partikel sind zumindest überwiegend als Plättchen ausgebildet.

Für die Bestimmung der durchschnittlichen Partikelgröße werden bei kugelförmigen Partikeln die Durchmesser herangezogen, bei Plättchen oder Stäbchen wird wie erwähnt der größte Durchmesser der Partikel (z. B. die Länge bei Stäbchen oder eine maximale Diagonale bei Plättchen) gemessen.

Besonders bevorzugt ist es, dass die Partikel ausschließlich als Plättchen ausgebildet sind. Die Plättchen richten sich im Wesentlichen mit einer Plättchenebene parallel zur Oberfläche des Gewebes aus. Dadurch ist es möglich, dass auch bei geringen Volumenanteilen bzw. mit dünnen Plättchen allfällig gebildete Abscheidungen gut kaschiert werden. Die Plättchen können dabei insbesondere aus einem Metall oder einer Legierung bestehen, beispielsweise einer sogenannten Silberbronze. Sind die Partikel aus einem Metall oder eine Legierung gebildet, ergibt sich auch eine gewisse Reflektion des Lackes, was zusätzlich dazu beiträgt, dass allfällig gebildete Abscheidungen nicht sichtbar sind und sich eine Farbe der hellen oder insbesondere weißen Membran bzw. des flächigen Gebildes auch im Laufe der Zeit bei Außenbetrachtung nicht ändert.

Das flächige Gebilde kann ein Flächengewicht von 750 g/m² bis 1600 g/m² aufweisen.

Das Gewebe des flächigen Gebildes, das den Kern einer Membran bildet, ist bevorzugt aus Multifilamenten gebildet. Hierbei kommen insbesondere Multifilamente aus organischen Materialen wie Polyacrylnitril oder einem Polyester gebildet sein. Das Gewebe selbst weist bevorzugt ein Flächengewicht von 200 g/m² bis 700 g/m² auf. Entsprechend den vorstehend dargestellten Vorteilen findet ein flächiges Gewebe insbesondere Verwendung für einen Biogasspeicher. Dabei kann das flächige Gewebe als Membran für die Dachkonstruktion eines Biogasspeichers eingesetzt werden. Möglich ist es auch, dass eine entsprechende Membran weitgehend eine äußere Hülle eines Biogasspeichers darstellt.

Gemäß den vorstehenden Erläuterungen ist ein Biogasspeicher vorzugsweise mit einer ein inneres Volumen umgebenden Außenfläche ausgebildet, die zumindest teilweise durch ein erfindungsgemäßes flächiges Gebilde gebildet ist, wobei die Lackschicht außenseitig angeordnet ist.

Weitere Merkmale, Vorteile und Wirkungen der Erfindung ergeben sich aus dem nachfolgend dargestellten Ausführungsbeispiel. In den Zeichnungen, auf welche dabei Bezug genommen wird, zeigen:
Fig. 1 eine schematische Darstellung eines Biogasspeichers;
Fig. 2 eine schematische Darstellung eines Querschnitts eines flächigen Gebildes.

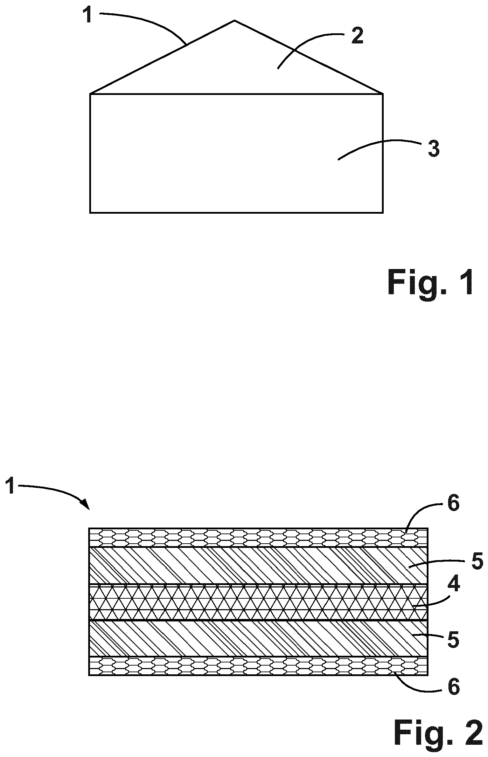

In Fig. 1 ist in stark schematisierter Darstellung ein Biogasspeicher 3 dargestellt. Der Biogasspeicher 3 umfasst eine beispielsweise in Draufsicht rund ausgebildete Behälterwand, auf welcher eine Dachkonstruktion 2 aufgesetzt ist. Die Dachkonstruktion 2 besteht aus einem flächigen Gebilde 1 bzw. einer Membran, die flexibel auf die Erhöhung eines Druckes im inneren Volumen des Biogasspeichers 3 reagieren bzw. sich ausdehnen kann. Möglich ist es aber auch, dass unterhalb der der Dachkonstruktion 2, welche von der Membran aufgespannt wird, eine Innenmembran vorgesehen ist. In diesem Fall ist die außenseitige Membran, welche die Dachkonstruktion 2 bildet oder zumindest deren wesentlichen Bestandteil darstellt, weitgehend unabhängig vom Innendruck im Biogasspeicher 3 und erfüllt vornehmlich die Funktion eines Schutzes der darunterliegenden Membran bzw. schützt vor der Witterung und anderen Einflüssen wie Sonneneinstrahlung.

Für die Dachkonstruktion 2 oder gegebenenfalls auch eine gesamte Außenhülle des Biogasspeichers 3 kommt das flächige Gebilde 1 zum Einsatz, welches in einem ausschnittsweisen Querschnitt in Fig. 2 dargestellt ist. Das flächige Gebilde 1 ist mehrlagig aufgebaut. Eine innere Schicht ist aus einem Gewebe 4 gebildet. Das Gewebe 4 selbst besteht in der Regel aus Multifilamenten, die aus Polyacrylnitril oder Polyester gebildet sein können. Möglich ist es auch, dass Gewebe 4 zum Einsatz kommen, wobei Kettfäden und Schussfäden aus einem unterschiedlichen Material bestehen. Bevorzugt werden synthetische organische Fasern bzw. entsprechend gebildete Multifilamente, zumal eine Membran aus dem flächigen Gebilde 1 vornehmlich dem Zweck dient, ein darunter befindliches Volumen des Biogasspeichers 3 gegen Umweltweinflüsse, aber auch gegen die Witterung zu schützen. Das Gewebe 4 weist beispielsweise ein Flächengewicht von 200 g/m² bis 700 g/m² auf. Das Gewebe 4 kann insbesondere ungefärbt vorliegen, also weiß erscheinen. Das Gewebe 4 ist zumindest nach außen hin von einer weiteren Schicht umgeben, die aus einem Kunststoff besteht. Eine entsprechende Kunststoffschicht 5, wie diese in Fig. 2 dargestellt ist, kann aus einem beliebigen Kunststoff relativ heller Farbe, insbesondere weißer Farbe, aber auch grauer oder (hell-)grüner Farbe gebildet sein. Bevorzugt besteht eine entsprechende Kunststoffschicht 5 aus PVC. Das PVC kann mit Stabilisatoren versehen sein, welche eine Hitzebeständigkeit, vor allem aber auch eine UV-Beständigkeit erhöhen. Wie in Fig. 2 dargestellt, kann eine Kunststoffschicht 5 zu beiden Seiten des Gewebes 4 angeordnet bzw. auf dem Gewebe 4 angebracht sein. Wenngleich in Fig. 2 die Schichten des Gewebes 4 und die Kunststoffschichten 5 als perfekte Ebenen dargestellt sind, versteht es sich, dass in der Praxis das Gewebe 4 eine gewisse Wellung aufweist und eine Dicke der Kunststoffschichten je nach Welligkeit des Gewebes 4 lokal variieren kann. Im Durchschnitt weisen die Kunststoffschichten 5 jedoch eine Dicke von etwa 200 µm bis 500 µm auf. Die Kunststoffschichten 5 sind wiederum jeweils von einer Lackschicht 6 umgeben. Bei einem Einsatz ist die in Fig. 2 obere Lackschicht 6 nach außen hin abschließend, bildet also bei einem Biogasspeicher 3 gemäß Fig. 1 eine für einen außenstehenden Betrachter sichtbare Oberfläche. Diese obere Lackschicht 6 zeichnet sich dadurch aus, dass in dieser neben dem Harz des Lackes zusätzlich Partikel wie plättchenförmige Aluminiumflitter oder dergleichen vorliegen bzw. dem aufzubringenden Lack beigemengt sind. Die Partikel weisen dabei eine durchschnittliche Partikelgröße von mehr als 5 µm auf. Ein Partikelgehalt kann variieren, beträgt allerdings mehr als 5 Gew.-%. Durch diese Partikel, die den Lack vor der Erstellung der oberen Lackschicht 6 beigegeben werden, wird effektiv verhindert, dass sich im laufenden Betrieb des Biogasspeichers 3 innerhalb der Membran, insbesondere am Gewebe 4 bildende Ausscheidungen dazu führen, dass sich die ins Weiße gehende äußere Oberfläche einer Membran nachteilig durchgehend oder, was noch weniger gewünscht ist, lediglich bereichsweise dunkel verfärbt. Ein Feststoffgehalt im Lack mit den angegebenen durchschnittlichen Partikelgrößen kann dabei mit Vorteil etwa im Bereich von 12 Gew.-% bis 20 Gew.-% liegen.

Zur Ermittlung möglicher Materialkombinationen zur Erzielung des gewünschten Effektes wurden verschiedene Versuche bei einem Schichtaufbau gemäß Fig. 2 durchgeführt, und zwar aufgehend von einem Gewebe 4 aus Multifilamenten, beschichtet mit Kunststoffschichten 5 aus PVC und einem herkömmlichen Klarlack für die Lackschichten 6 mit einzelnen Variationen wie folgt:
Beispiel 1: Verwendung eines Low-Wick-Garns - keine Wirksamkeit;
Beispiel 2: Verwendung eines Polyvinylidenchlorid-Lacks, Molmassenverteilung 1, außenseitig - geringe Wirksamkeit;
Beispiel 3: Verwendung eines Polyvinylidenchlorid-Lacks, Molmassenverteilung 2 - geringe Wirksamkeit;
Beispiel 4: Verwendung eines Polyvinylidenchlorid-Lacks, Molmassenverteilung 3, außenseitig - mittlere Wirksamkeit;
Beispiel 5: Verwendung eines Polyvinylidenchlorid-Lacks, Molmassenverteilung 1, innenseitig - keine Wirksamkeit;
Beispiel 6: Verwendung eines Polyacryl-Lacks mit Silberbronze, innenseitig - keine Wirksamkeit;
Beispiel 7: Verwendung eines Polyacryl-Lacks mit Silberbronze, außenseitig - Wirksamkeit über zwei Jahre geprüft und bestätigt;
Beispiel 8: Verwendung einer geschäumten PVC-Variante als Kunststoffschicht 6 - keine Wirksamkeit;
Beispiel 9: Verwendung einer zusätzlichen Lackzwischenschicht - keine Wirksamkeit.

Wie aus den vorstehenden Versuchen ersichtlich ist, führt lediglich eine Materialkombination gemäß Beispiel 7 zu den gewünschten Wirkungen. Die Erklärung könnte hierin liegen, dass die beigemengten Partikel bzw. die Silberbronze dazu führt, dass aufgrund der dann veränderten Eigenschaften der äußeren Lackschicht 6 in Bezug auf Reflektion und Deckkraft zwar nicht die Bildung von darunterliegenden Abscheidungen verhindert werden kann, die Abscheidungen allerdings keine optische Wirksamkeit mehr entfalten.

Eine Membran wie vorstehend beschrieben kann für beliebige Elemente eines Biogasspeichers 3 eingesetzt werden, z. B. für die gesamte Außenhülle oder nur eine Dachkonstruktion 2. Auch eine Verwendung für Dächer von Hochsilos ist möglich.

## Patentansprüche

1. Flächiges Gebilde (1), insbesondere für eine Dachkonstruktion (2) eines Biogasspeichers (3), umfassend ein Gewebe (4), welches auf zumindest einer Seite zumindest bereichsweise von einer Kunststoffschicht (5) umgeben ist, wobei auf der Kunststoffschicht (5) eine Lackschicht (6) aufgebracht ist, wobei die Lackschicht (6) neben einem Lackharz beigemengte Partikel enthält, **dadurch gekennzeichnet, dass** eine durchschnittliche Partikelgröße der Partikel in der Lackschicht (6) mehr als 5 µm beträgt und die Lackschicht (6) mehr als 5 Gew.-% Partikel enthält, wobei die Partikel zumindest überwiegend als Plättchen ausgebildet sind.

2. Flächiges Gebilde (1) nach Anspruch 1, **dadurch gekennzeichnet, dass** das Gewebe (4) beidseitig von einer Kunststoffschicht (5), vorzugsweise aus demselben Material, umgeben ist.

3. Flächiges Gebilde (1) nach Anspruch 2, **dadurch gekennzeichnet, dass** auf jeder Kunststoffschicht (5) eine Lackschicht (6) aufgebracht ist.

4. Flächiges Gebilde (1) nach einem der Ansprüche 1 bis 3, **dadurch gekennzeichnet, dass** die Lackschicht (6) mehr als 10 Gew.-% Partikel, insbesondere 12 Gew.-% bis 20 Gew.-% Partikel, enthält.

5. Flächiges Gebilde (1) nach einem der Ansprüche 1 bis 4, **dadurch gekennzeichnet, dass** die durchschnittliche Partikelgröße der Partikel in der Lackschicht (6) mehr als 10 µm, insbesondere 12 µm bis 25 µm, beträgt.

6. Flächiges Gebilde (1) nach Anspruch 1, **dadurch gekennzeichnet, dass** die Plättchen aus einem Metall oder einer Legierung bestehen.

7. Flächiges Gebilde (1) nach einem der Ansprüche 1 bis 6, **dadurch gekennzeichnet, dass** das Gebilde (1) ein Flächengewicht von 750 g/m² bis 1600 g/m² aufweist.

8. Flächiges Gebilde (1) nach einem der Ansprüche 1 bis 7, **dadurch gekennzeichnet, dass** das Gewebe (4) aus Multifilamenten gebildet ist.

9. Flächiges Gebilde (1) nach Anspruch 8, **dadurch gekennzeichnet, dass** das Gewebe (4) ein Flächengewicht von 200 g/m² bis 700 g/m² aufweist.

10. Verwendung eines flächigen Gebildes (1) nach einem der Ansprüche 1 bis 9 für einen Biogasspeicher (3).

11. Biogasspeicher (3) mit einer ein inneres Volumen umgebenden Außenfläche, die zumindest teilweise durch ein flächiges Gebilde (1) nach einem der Ansprüche 1 bis 9 gebildet ist, wobei die Lackschicht (6) außenseitig angeordnet ist.

## Claims

1. A flat article (1), in particular for a roof structure (2) of a biogas store (3), comprising a fabric (4), which is surrounded on at least one side at least in regions by a plastics layer (5), wherein a coating layer (6) is applied to the plastics layer (5), wherein the coating layer (6), besides a coating resin, also contains admixed particles, **characterised in that** an average particle size of the particles in the coating layer (6) is more than 5 µm and the coating layer (6) contains more than 5 % by weight of particles, wherein the particles are formed at least predominantly as platelets.

2. The flat article (1) according to claim 1, **characterised in that** the fabric (4) is surrounded on both sides by a plastics layer (5), preferably made of the same material.

3. The flat article (1) according to claim 2, **characterised in that** a coating layer (6) is applied to each plastics layer (5).

4. The flat article (1) according to any one of claims 1 to 3, **characterised in that** the coating layer (6) contains more than 10 % by weight of particles, in particular 12 % by weight to 20 % by weight of particles.

5. The flat article (1) according to any one of claims 1 to 4, **characterised in that** the average particle size of the particles in the coating layer (6) is more than 10 µm, in particular 12 µm to 25 µm.

6. The flat article (1) according to claim 1, **characterised in that** the platelets consist of a metal or an alloy.

7. The flat article (1) according to any one of claims 1 to 6, **characterised in that** the article (1) has a mass per unit area of 750 g/m² to 1600 g/m².

8. The flat article (1) according to any one of claims 1 to 7, **characterised in that** the fabric (4) is formed from multi-filaments.

9. The flat article (1) according to claim 8, **characterised in that** the fabric (4) has a mass per unit area of 200 g/m² to 700 g/m².

10. Use of a flat article (1) according to any one of claims 1 to 9 for a biogas store (3).

11. A biogas store (3) with an outer surface which surrounds an inner volume and which is formed at least in part by a flat article (1) according to any one of claims 1 to 9, wherein the coating layer (6) is arranged on the outer side.

## Revendications

1. Structure bidimensionnelle (1), destinée notamment à la toiture (2) d'un réservoir de biogaz (3), comprenant un tissu (4) qui est entouré, au moins sur l'une de ces faces, au moins sur certaines parties d'une couche de matière plastique (5), la couche de matière plastique étant revêtue d'une couche de vernis (6), la couche de vernis (6) contenant outre la résine formant le vernis des particules ajoutées, **caractérisée en ce que** la granulométrie moyenne des particules au sein de la couche de vernis (6) est supérieure à 5 µm et la couche de vernis (6) contient plus de 5 % en poids de particules, lesdites particules ayant au moins majoritairement la forme de microplaquettes.

2. Structure bidimensionnelle (1) selon la revendication 1, **caractérisée en ce que** le tissu (4) est entouré, sur ses deux faces, de couches de matière plastique (5) qui sont préférentiellement formées d'un même type de matériau.

3. Structure bidimensionnelle (1) selon la revendication 2, **caractérisé en ce que** chacune des couches de matière plastique (5) est revêtue d'une couche de vernis (6).

4. Structure bidimensionnelle (1) selon l'une des revendications 1 à 3, **caractérisée en ce que** la couche de vernis (6) contient plus de 10 % en poids de particules, notamment 12 % en poids à 20 % en poids de particules.

5. Structure bidimensionnelle (1) selon l'une des revendications 1 à 4, **caractérisée en ce que** la granulométrie moyenne des particules au sein de la couche de vernis (6) est supérieure à 10 µm, notamment comprise entre 12 µm et 25 µm.

6. Structure bidimensionnelle (1) selon la revendication 1, **caractérisée en ce que** lesdites microplaquettes sont constituées d'un métal ou d'un alliage.

7. Structure bidimensionnelle (1) selon l'une des revendications 1 à 6, **caractérisée en ce que** la structure (1) présente une masse surfacique comprise entre 750 g/m² et 1 600 g/m².

8. Structure bidimensionnelle (1) selon l'une des revendications 1 à 7, **caractérisée en ce que** le tissu (4) est formé de multifilaments.

9. Structure bidimensionnelle (1) selon la revendication 8, **caractérisée en ce que** le tissu (4) présente une masse surfacique comprise entre 200 g/m² et 700 g/m².

10. Utilisation d'une structure bidimensionnelle (1) selon l'une des revendications 1 à 9 pour un réservoir de biogaz (3).

11. Réservoir de biogaz (3) comportant une surface extérieure qui entoure un volume intérieur et qui est formée, au moins partiellement, par une structure bidimensionnelle (1) selon l'une des revendications 1 à 9, la couche de vernis (6) étant disposée à l'extérieur.
